# EUROPEAN PATENT SPECIFICATION

(11) **EP 3 479 684 B1**
(45) Date of publication and mention of the grant of the patent: **23.09.2020**
(21) Application number: 18200996.9
(22) Date of filing: 17.10.2018
(51) Int. Cl.: A01G 31/00, A01G 22/25

(54) **METHOD FOR HYDROPONIC CULTURE OF PLANT OF FAMILY ASTERACEAE**
VERFAHREN ZUR HYDROPONISCHEN KULTUR VON PFLANZEN DER FAMILIE ASTERACEAE
PROCÉDÉ DE CULTURE HYDROPONIQUE DE PLANTES DE LA FAMILLE DES ASTERACEAE

(30) Priority: 06.11.2017 JP 2017213770
(43) Date of publication of application: 08.05.2019
(73) Proprietor: Sumitomo Rubber Industries, Ltd., Kobe-shi, Hyogo 651-0072 (JP)
(72) Inventor: INOUE, Yukino, Kobe-shi, Hyogo 651-0072 (JP); YAMAGUCHI, Haruhiko, Kobe-shi, Hyogo 651-0072 (JP); MIYAJI, Daiki, Kobe-shi, Hyogo 651-0072 (JP)
(74) Representative: TBK

(56) References cited:
- CN-U- 204 811 214
- JP-A- 2010 142 173
- US-A1- 2011 232 190
- US-A1- 2016 237 254
- US-A1- 2017 231 162
- US-B2- 6 807 770

## Description

### TECHNICAL FIELD

The present invention relates to a method for hydroponic culture of a plant of the family *Asteraceae.*

### BACKGROUND ART

Natural rubber industrially used currently is derived from *Hevea brasiliensis* (Para rubber tree). However, since the region where *Hevea brasiliensis* can be cultivated is limited, there is a high demand for alternative plants producing natural rubber.

As an alternative plant producing natural rubber, *Taraxacum kok-saghyz* (Russian dandelion) has been drawing attention because natural rubber can be harvested from its roots, and even temperate zones can selected to cultivate it. Accordingly, methods for extracting rubber from this plant have been under development. Moreover, other methods have been proposed such as bringing *Taraxacum kok-saghyz* into contact with jasmonic acid or a derivative thereof to promote latex production, thereby increasing latex yield (see, for example, Patent Literature 1).

### CITATION LIST

### PATENT LITERATURE

Patent Literature 1: JP 2010-142173 A

US 2016/237254 A1 describes a method for extracting latex from a plant of the Asteraceae family and a method for cultivating a plant of the Asteraceae family, which allow such plants to be reused. The method for extracting latex included a cutting step of cutting roots of a plant of the Asteraceae family in such a manner that the roots are partly left on the plant.

US 2017/231162 A1 describes a method for cultivating plants of the Asteraceae family, which promotes flower bud formation in the reproductive phase, wherein the cultivation temperature in the reproductive phase is set lower by at least 5° C than the cultivation temperature in the vegetative phase to promote flower bud formation in the reproductive phase.

US 2011/232190 A1 describes a hydroponic system comprising an apparatus for growing plants hydroponically. The apparatus comprises a container defining a roughly annular trough or cavity for holding nutrient fluid, and a plate member received on the container supporting a plurality of baskets holding root portions of plants therein such that the baskets are accommodated in the trough for communication with the nutrient fluid. The baskets are adapted to rotate about axes thereof. A nutrient feeding mechanism comprises a feeding pump configured within a nutrient fluid storing reservoir tank and adapted to deliver nutrient fluid via a supply tube to the container. The apparatus comprises a light, a roof mounted on a support for supporting the container above a surface, and a plurality of door panels movably configured between the roof and the container. Air feeding means feed air to a chamber defined by the base, roof and doors.

US 6 807 770 B2 describes an aeroponic growing apparatus which comprises a low pressure liquid nutrient delivery system generated by centrifugal force utilizing a rotating cylinder device. The rotating cylinder device distributes liquid nutrient solution to the roots of plants by use of centrifugal force. The geometrical shape of the enclosed root growth chamber allows for fractionated droplets to ricochet in multiple random directions thus completely surrounding the plant roots in 360° in any plane.

### SUMMARY OF INVENTION

### TECHNICAL PROBLEM

Natural rubber can be harvested from the roots of *Taraxacum kok-saghyz.* When natural rubber is harvested from the roots of *Taraxacum kok-saghyz* by cutting its fine roots, only a little amount of the rubber component can be collected. This means that the size (thickness) of the roots greatly affects the yield of rubber. Hence, in order to efficiently collect rubber from *Taraxacum kok-saghyz,* it is necessary to culture it to thicken its roots.

However, adequate studies have not been done on culturing methods suitable for growing the roots of plants of the family *Asteraceae* such as *Taraxacum kok-saghyz,* and there is room for further investigation.

An object of the present invention is to solve the problem and provide a method for hydroponic culture of a plant of the family *Asteraceae* which is suitable for growing the roots of the plant of the family *Asteraceae.*

### SOLUTION TO PROBLEM

The present inventors have made extensive studies on culturing methods suitable for growing the roots of plants of the family *Asteraceae,* on which no adequate studies have been done, and as a result, have found that when hydroponic culture is performed by culturing a plant of the family *Asteraceae* while adjusting the amount of a nutrient solution so that 10 to 50% of the total length from the tip of the tap root of the plant of the family *Asteraceae* is soaked in the nutrient solution, and the rest of the tap root is exposed to a gas, the growth of the tap root can be promoted to form a long, thick tap root, and further rubber synthesis can be activated, whereby the weight of rubber accumulated in the roots can be increased. This finding has led to the completion of the present invention.

Specifically, the present invention relates to a method for hydroponic culture of a plant of the family *Asteraceae,* the method including a hydroponic culture step including culturing a plant of the family *Asteraceae* while adjusting the amount of a nutrient solution so that 10 to 50% of the total length from the tip of the tap root of the plant of the family *Asteraceae* is soaked in the nutrient solution, and the rest of the tap root is exposed to a gas.

The plant of the family *Asteraceae* is preferably *Taraxacum kok-saghyz* or *Taraxacum brevicorniculatum.*

The hydroponic culture step is preferably carried out at a culture temperature of 25°C or lower.

The hydroponic culture step preferably includes light conditions including a photoperiod of 15 hours or longer and an illuminance at leaf level of 6,000 lx or higher.

### ADVANTAGEOUS EFFECTS OF INVENTION

The method for hydroponic culture of a plant of the family *Asteraceae* according to the present invention includes hydroponically culturing a plant of the family *Asteraceae* while adjusting the amount of a nutrient solution so that 10 to 50% of the total length from the tip of the tap root of the plant of the family *Asteraceae* is soaked in the nutrient solution, and the rest of the tap root is exposed to a gas, in order to promote the growth of the tap root to form a long, thick tap root, and further to activate rubber synthesis, thereby increasing the weight of rubber accumulated in the roots.

### DESCRIPTION OF EMBODIMENTS

The method for hydroponic culture of a plant of the family *Asteraceae* according to the present invention includes hydroponically culturing a plant of the family *Asteraceae* while adjusting the amount of a nutrient solution so that 10 to 50% of the total length from the tip of the tap root of the plant of the family *Asteraceae* is soaked in the nutrient solution, and the rest of the tap root is exposed to a gas, in order to promote the growth of the tap root to form a long, thick tap root, and further to activate rubber synthesis, thereby increasing the weight of rubber accumulated in the roots.

Branched roots may be formed in hydroponic culture. According to the present invention, however, it is possible to promote the growth of the tap root in the tip direction and inhibit the formation of unnecessary branched roots by performing hydroponic culture under conditions where the tip portion of the tap root (10 to 50% of the total length from the tip of the tap root) is soaked in a nutrient solution, and the rest of the tap root, including the base, is not soaked in the nutrient solution but is exposed to a gas (air). Moreover, in the method for hydroponic culture of the present invention, since the roots contact the nutrient solution and gas (air), there are no physical obstacles in the growing direction of the roots, and therefore there will not be any branched root, which can be caused by the presence of a hard obstacle in the growing direction of the roots. Furthermore, when the nutrient solution is a sterilized or disinfected nutrient solution, the risk of root diseases can be reduced.

Also, in the method for hydroponic culture of the present invention in which hydroponic culture is performed under conditions where the tip portion of the tap root (10 to 50% of the total length from the tip of the tap root) is soaked in a nutrient solution, and the rest of the tap root is not soaked in the nutrient solution but is exposed to a gas (air), root respiration can be promoted to prevent root rot.

Moreover, adjusting the amount of the nutrient solution is sufficient to perform hydroponic culture under conditions where the tip portion of the tap root (10 to 50% of the total length from the tip of the tap root) is soaked in the nutrient solution, and the rest of the tap root is exposed to a gas (air). Thus, it is unnecessary to prepare a special apparatus as used in, for example, a spraying method in which a nutrient solution is sprayed as a mist during culture, or a microbubble method in which microbubbles are supplied during hydroponic culture. Furthermore, since it is not required to dig up soil as in soil culture, the roots are easy to pull out and less likely to be damaged in some parts during harvesting. In addition, since the process of root growth is visible, it is possible to thin out stunted individuals in advance.

As described above, the present invention uses a simple process of adjusting the amount of the nutrient solution in hydroponic culture in order to promote the growth of the tap root of a plant of the family *Asteraceae* to form a long, thick tap root, and further to activate rubber synthesis, thereby increasing the weight of rubber accumulated in the roots.

Examples of plants of the family *Asteraceae* to which the method of the present invention can be applied include, but are not limited to, plants belonging to the genera *Sonchus, Solidago, Helianthus, Taraxacum,* and *Lactuca.*

Examples of the plants of the genus *Sonchus* include *Sonchus oleraceus, Sonchus asper, Sonchus brachyotus,* and *Sonchus arvensis.*

Examples of the plants of the genus *Solidago* include *Solidago altissima, Solidago virgaurea* subsp. *asiatica, Solidago virgaurea* subsp. *leipcarpa, Solidago virgaurea* subsp. *leipcarpa* f. *paludosa, Solidago virgaurea* subsp. *gigantea,* and *Solidago gigantea* Ait. var. *leiophylla* Fernald.

Examples of the plants of the genus *Helianthus* include *Helianthus annuus, Helianthus argophyllus, Helianthus atrorubens, Helianthus debilis, Helianthus decapetalus,* and *Helianthus giganteus.*

Examples of the plants of the genus *Taraxacum* include dandelion (Taraxacum), *Taraxacum venustum* H. Koidz, *Taraxacum hondoense* Nakai, *Taraxacum platycarpum* Dahlst, *Taraxacum japonicum, Taraxacum officinale* Weber, *Taraxacum kok-saghyz,* and *Taraxacum brevicorniculatum.*

Examples of the plants of the genus *Lactuca* include *Lactuca sativa* and *Lactuca indica.*

The method of the present invention is suitably applicable to plants of the genus *Taraxacum,* among others, and is more suitably applicable to *Taraxacum kok-saghyz* or *Taraxacum brevicorniculatum.*

The method for hydroponic culture of a plant of the family *Asteraceae* according to the present invention may include any other step as long as it includes the hydroponic culture step.

The plant of the family *Asteraceae* to be subjected to the hydroponic culture step in the present invention may be one in a stage usually used for hydroponic culture. For example, young plants obtained after seed dormancy breaking and subsequent rooting may be subjected to the hydroponic culture step.

The seeds may be genetically modified seeds or non-genetically modified seeds. Moreover, steps for preparing such young plants, including seeding (seeding step), breaking dormancy (dormancy-breaking step), and rooting (rooting step), may each be performed in a manner conventionally used for the species of the family *Asteraceae* of interest.

The hydroponic culture step in the present invention may be carried out in any manner as long as a plant of the family *Asteraceae* is cultured while adjusting the amount of a nutrient solution so that 10 to 50% of the total length from the tip of the tap root of the plant of the family *Asteraceae* is soaked in the nutrient solution, and the rest of the tap root is exposed to a gas (air). However, it is preferred that during the period of hydroponic culture, the plant is substantially always cultured under conditions where 10 to 50% of the total length from the tip of the tap root is soaked in the nutrient solution, and the rest of the tap root is exposed to a gas (air).

The term "tap root" herein refers to a root which is developed from a primary root formed directly from an embryo and whose thickness at the base is the largest.

The term "substantially always (during the period of hydroponic culture)" herein means 70% or longer, preferably 80% or longer, more preferably 90% or longer, still more preferably 95% or longer, most preferably 100% of the length of the period of hydroponic culture.

The phrase "10 to 50% of the total length from the tip of the tap root of the plant of the family *Asteraceae* is soaked in the nutrient solution" herein means that a length from the tip of the tap root that is 10 to 50% of the total length from the base of the tap root to the tip of the tap root of the plant of the family *Asteraceae* is soaked in the nutrient solution. In the present invention, the amount of the nutrient solution is adjusted on the basis of the length of the tap root.

Moreover, adjusting the amount of the nutrient solution is sufficient to perform hydroponic culture under conditions where 10 to 50% of the total length from the tip of the tap root of the plant of the family *Asteraceae* is soaked in the nutrient solution, and the rest of the tap root is exposed to a gas (air). The method for adjusting the amount of the nutrient solution is not particularly limited, and the nutrient solution may be appropriately added or removed in a conventional manner.

In the hydroponic culture step of the present invention which includes culturing a plant of the family *Asteraceae* while adjusting the amount of a nutrient solution so that 10 to 50% of the total length from the tip of the tap root of the plant of the family *Asteraceae* is soaked in the nutrient solution, and the rest of the tap root is exposed to a gas (air), the length of the tap root soaked in the nutrient solution is preferably 20% or more, more preferably 25% or more, but preferably 40% or less, more preferably 35% or less, of the total length from the tip.

The gas may be any gas that can be used for respiration of the roots of the plant of the family *Asteraceae,* i.e., any gas containing oxygen at an appropriate concentration, such as, typically, air.

The nutrient solution is prepared by dissolving in water a fertilizer containing nutrients required for the plant to grow, and may be any nutrient solution suitable for the growth of the species of the family *Asteraceae* of interest. Any conventionally known nutrient solution may be used including, for example, the KANEKO fertilizer for soilless culture "Farm Ace No. 1" (produced by KANEKO SEEDS CO., LTD.), the KANEKO fertilizer for soilless culture "Farm Ace No. 2" (produced by KANEKO SEEDS CO., LTD.), the fertilizer for soilless culture "OAT House No. 1" (produced by OAT Agrio Co., Ltd.), and the fertilizer for soilless culture "OAT House No. 2" (produced by OAT Agrio Co., Ltd.).

For example, OAT House No. 1 is a powdery fertilizer containing 10.0% by mass of total nitrogen, 8.0% by mass of water-soluble phosphate, 27.0% by mass of water-soluble potassium, 4.0% by mass of water-soluble magnesium, 0.10% by mass of water-soluble manganese, 0.10% by mass of water-soluble boron, 0.18% by mass of iron, 0.002% by mass of copper, 0.006% by mass of zinc, and 0.002% by mass of molybdenum.

Moreover, OAT House No. 2 is a powdery fertilizer containing 11.0% by mass of total nitrogen and 23.0% by mass of lime.

For example, when OAT House No. 1 and OAT House No. 2 are used to prepare a nutrient solution, a known formulation (e.g. formulation A or C) may be used, according to which OAT House No. 1 may be dissolved in water, and OAT House No. 2 may be added and dissolved into the resulting solution.

The composition of formulation A made from OAT House No. 1 (produced by OAT Agrio Co., Ltd.) and OAT House No. 2 (produced by OAT Agrio Co., Ltd.) is listed below by way of example.
Total nitrogen (TN): 260 ppm
(in detail, ammonia nitrogen (AN): 23 ppm, nitrate nitrogen (NN): 233 ppm)
Phosphate (P₂O₅): 120 ppm
Potassium (K₂O): 405 ppm
Lime (CaO): 230 ppm
Magnesium (MgO): 60 ppm
Manganese (MnO): 1.5 ppm
Boron (B₂O₃): 1.5 ppm
Iron (Fe): 2.7 ppm
Copper (Cu): 0.03 ppm
Zinc (Zn): 0.09 ppm
Molybdenum (Mo): 0.03 ppm
EC value (dS/m): 2.6

The water-soluble phosphate content of the nutrient solution is preferably 90 to 200 ppm. A water-soluble phosphate content in this range can allow the roots of the plant of the family *Asteraceae* to grow particularly well. The water-soluble phosphate content is more preferably 100 to 160 ppm, still more preferably 110 to 140 ppm.

The water-soluble potassium content of the nutrient solution is preferably 350 to 500 ppm. A water-soluble potassium content in this range can allow the roots of the plant of the family *Asteraceae* to grow particularly well. The water-soluble potassium content is more preferably 360 to 490 ppm, still more preferably 400 to 485 ppm.

The lime content of the nutrient solution is preferably 150 to 300 ppm. A lime content in this range can allow the roots of the plant of the family *Asteraceae* to grow particularly well. The lime content is more preferably 160 to 250 ppm, still more preferably 180 to 230 ppm.

The water-soluble magnesium content of the nutrient solution is preferably 30 to 200 ppm. The enzyme activity of cis-prenyltransferase, which is involved in rubber synthesis, requires divalent cations, and magnesium ions are believed to be particularly suitable. Thus, it is important to supply magnesium ions for rubber synthesis. On the other hand, excessive supply of magnesium ions is believed to inhibit absorption of another nutrient, calcium ions, in some cases. In these circumstances, when the water-soluble magnesium content falls within the above range, the roots of the plant of the family *Asteraceae* can be allowed to grow particularly well, and rubber synthesis can be further activated. The water-soluble magnesium content is more preferably 40 to 150 ppm, still more preferably 50 to 100 ppm.

The pH of the nutrient solution is preferably 4.0 to 7.0. A pH in this range can allow the roots of the plant of the family *Asteraceae* to grow particularly well. The pH is more preferably 5.0 to 6.5.

The temperature of the nutrient solution in the hydroponic culture step is preferably 25°C or lower, more preferably 23°C or lower, but is preferably 20°C or higher, more preferably 22°C or higher. A nutrient solution temperature in the hydroponic culture step that falls within such a range can provide good growth without causing growth failure.

The illuminance at leaf level in the hydroponic culture step is preferably 6,000 lx or higher, more preferably 6,500 lx or higher, still more preferably 7,000 lx or higher, but is preferably 20,000 lx or lower, more preferably 18,000 lx or lower, still more preferably 15,000 lx or lower, further preferably 12,000 lx or lower, particularly preferably 10,000 lx or lower. An illuminance at leaf level in the hydroponic culture step that falls within such a range can provide good growth without causing photoinhibition.

In the present invention, the illuminance at leaf level is measured in accordance with JIS C 7612.

The light source used to achieve the above illuminance is not particularly limited, and natural light or artificial lights, or a combination of them may be used. When artificial light is used, it may be, for example, a light-emitting diode (LED), a halogen lamp, an incandescent lamp, a fluorescent lamp, an arc lamp, an electrodeless discharge lamp, a low-pressure discharge lamp, a cold cathode fluorescent tube, an external electrode fluorescent lamp, an electroluminescent lamp, or an HID lamp. Examples of the HID lamp include high-pressure mercury lamps, metal halide lamps, and high-pressure sodium lamps. These light sources may be used alone or in combinations of two or more.

The photoperiod in the hydroponic culture step is preferably 15 hours or longer, more preferably 16 hours or longer. Although the upper limit of the photoperiod is not particularly critical, it is preferably 22 hours or shorter, more preferably 20 hours or shorter. A photoperiod in the hydroponic culture step that falls within such a range can provide good growth without causing growth failure.

The culture temperature in the hydroponic culture step is preferably 25°C or lower, more preferably 23°C or lower, but is preferably 20°C or higher, more preferably 22°C or higher. A culture temperature in the hydroponic culture step that falls within such a range can provide good growth without causing growth failure.

The culture period in the hydroponic culture step is preferably 1 month or longer, more preferably 3 months or longer, still more preferably 4 months or longer. A culture period in the hydroponic culture step that falls within such a range can provide sufficient growth to accumulate rubber in the roots without withering. The upper limit of the culture period is not particularly critical.

Other conditions for hydroponic culture are not particularly limited, and conventional conditions suitable for the growth of the species of the family *Asteraceae* of interest may be used for culture. Moreover, the apparatus or the like for hydroponic culture is not particularly limited, and any conventional apparatus or the like for hydroponic culture may be used.

### EXAMPLES

The present invention will be specifically described with reference to examples. However, the present invention is not limited only to these examples.

### (Example 1)

### [Seeding/dormancy-breaking/rooting/transplanting]

Two or three filter papers were stacked and placed on a petri dish and then moistened with deionized water. Seeds of *Taraxacum kok-saghyz* (available from United States Department of Agriculture, Animal and Plant Health Inspection Service, Plant Protection and Quarantine) were placed at intervals of 1.5 cm or more from each other on the filter papers. The petri dish was covered with a lid and sealed with a parafilm. The petri dish was placed in a refrigerator (4°C) for 3 days to break dormancy. After the dormancy-breaking treatment, the petri dish containing the seeds was placed in an incubator at 16 to 22°C.

### [Culturing]

After the seeds were rooted to a root length of 1 cm or longer, they were planted into a urethane mat for hydroponic culture. The nutrient solution used for hydroponic culture was formulation A made from OAT House No. 1 (produced by OAT Agrio Co., Ltd.) and OAT House No. 2 (produced by OAT Agrio Co., Ltd.).

The composition of formulation A made from OAT House No. 1 (produced by OAT Agrio Co., Ltd.) and OAT House No. 2 (produced by OAT Agrio Co., Ltd.) is listed below.
Total nitrogen (TN): 260 ppm
(in detail, ammonia nitrogen (AN): 23 ppm, nitrate nitrogen (NN): 233 ppm)
Phosphate (P₂O₅): 120 ppm
Potassium (K₂O): 405 ppm
Lime (CaO): 230 ppm
Magnesium (MgO): 60 ppm
Manganese (MnO): 1.5 ppm
Boron (B₂O₃): 1.5 ppm
Iron (Fe): 2.7 ppm
Copper (Cu): 0.03 ppm
Zinc (Zn): 0.09 ppm
Molybdenum (Mo): 0.03 ppm
EC value (dS/m): 2.6

Hydroponic culture was performed at 23°C under a cycle of 16 hours light and 8 hours dark for 5 months. The illuminance at leaf level under light conditions was maintained at 7,000 to 10,000 lx. The illuminance at leaf level under dark conditions was maintained at 1 lx or lower. During the hydroponic culture, the amount of the nutrient solution was adjusted so that 20 to 40% of the total length from the tip of the tap root of *Taraxacum kok-saghyz* under culture was substantially always soaked in the nutrient solution, and the rest of the tap root was exposed to air.

### [Measurement of thickness/length of tap root and weight of underground parts (total roots)]

The thickness and length of the tap root and the weight of the underground parts (total roots) of *Taraxacum kok-saghyz* were measured after the above-described 5-month hydroponic culture. The results are shown in Table 1.

### [Harvesting of rubber]

The roots (total roots) of *Taraxacum kok-saghyz* after the 5-month hydroponic culture were cut to harvest latex. The harvesting of latex was carried out by cutting the roots at intervals of about 5 mm starting from the tips of the roots until the length of the roots left on the plant reached 3.0 cm. Ethanol was added to the harvested latex, and the mixture was allowed to stand protected from light. The solution containing ethanol was removed from the latex solution obtained after ethanol immersion, and the residue was evaporated to dryness. Then, the weight of rubber was measured. The results are shown in Table 1.

### (Comparative Example 1)

Hydroponic culture was performed in the same manner as in Example 1, except that during the culture, the amount of the nutrient solution was adjusted so that 90 to 100% (i.e. total roots) of the total length from the tip of the tap root of *Taraxacum kok-saghyz* under culture was substantially always soaked in the nutrient solution, and the rest of the tap root was exposed to air. The thickness and length of the tap root and the weight of the underground parts (total roots) were measured, and rubber was harvested to measure the weight of rubber. The results are shown in Table 1.

### (Comparative Example 2)

### [Seeding/dormancy-breaking/rooting/transplanting]

Two or three filter papers were stacked and placed on a petri dish and then moistened with deionized water. Seeds of *Taraxacum kok-saghyz* (available from United States Department of Agriculture, Animal and Plant Health Inspection Service, Plant Protection and Quarantine) were placed at intervals of 1.5 cm or more from each other on the filter papers. The petri dish was covered with a lid and sealed with a parafilm. The petri dish was placed in a refrigerator (4°C) for 3 days to break dormancy. After the dormancy-breaking treatment, the petri dish containing the seeds was placed in an incubator at 16 to 22°C.

### [Culturing]

After the seeds were rooted to a root length of 1 cm or longer, they were planted in a pot containing vermiculite.

Culturing (soil culture) was performed at 23°C under a cycle of 16 hours light and 8 hours dark for 5 months (the total roots of *Taraxacum kok-saghyz* were buried in soil during the culture). The illuminance at leaf level under light conditions was maintained at 7,000 to 10,000 lx. The illuminance at leaf level under dark conditions was maintained at 1 lx or lower. During the culture, a 500 fold dilution of HYPONICA (produced by Kyowa Co., Ltd.) (liquid fertilizer) was appropriately supplied.

### [Measurement of thickness/length of tap root and weight of underground parts (total roots)]

The thickness and length of the tap root and the weight of the underground parts (total roots) of *Taraxacum kok-saghyz* were measured after the above-described 5-month soil culture. The results are shown in Table 1.

### [Harvesting of rubber]

The roots (total roots) of *Taraxacum kok-saghyz* after the 5-month soil culture were cut to harvest latex. The harvesting of latex was carried out by cutting the roots at intervals of about 5 mm starting from the tips of the roots until the length of the roots left on the plant reached 3.0 cm. Ethanol was added to the harvested latex, and the mixture was allowed to stand protected from light. The solution containing ethanol was removed from the latex solution obtained after ethanol immersion, and the residue was evaporated to dryness. Then, the weight of rubber was measured. The results are shown in Table 1.

**[Table 1]**

| | Example 1 | Comparative Example 1 | Comparative Example 2 |
|---|---|---|---|
| Thickness of tap root (mm) | 40.54 | 25.48 | 23.79 |
| Length of tap root (mm) | 120.7 | 91.2 | 82.4 |
| Weight of underground parts (g) | 29.70 | 24.20 | 20.28 |
| Weight of rubber (mg) | 114.3 | 27.0 | 10.5 |

As can be seen from Table 1, the thickness and length of the tap root and the weight of the underground parts (total roots) of Example 1 in which hydroponic culture was performed while adjusting the amount of a nutrient solution so that 10 to 50% of the total length from the tip of the tap root was soaked in the nutrient solution, and the rest of the tap root was exposed to a gas (air) were all greater than those of Comparative Example 1 in which the entire tap root was soaked in the nutrient solution during hydroponic culture, and Comparative Example 2 in which soil culture was performed. Moreover, the weight of rubber was also increased in Example 1. Since *Taraxacum kok-saghyz* plants accumulate rubber in their roots, the increase in the weight of rubber seems to be due to the increase in the thickness and length of the tap root and the weight of the total roots. Furthermore, the ratio of the weight of rubber to the weight of the roots (the weight of the underground parts) in Example 1 was higher than in Comparative Examples 1 and 2. This is probably because rubber synthesis was activated by performing hydroponic culture under conditions where 10 to 50% of the total length from the tip of the tap root was soaked in the nutrient solution, and the rest of the tap root was exposed to a gas (air).

Therefore, it was demonstrated that when a plant of the family *Asteraceae* is hydroponically cultured while adjusting the amount of a nutrient solution so that 10 to 50% of the total length from the tip of the tap root of the plant of the family *Asteraceae* is soaked in the nutrient solution, and the rest of the tap root is exposed to a gas, the growth of the tap root can be promoted to form a long, thick tap root, and further rubber synthesis can be activated, whereby the weight of rubber accumulated in the roots can be increased.

Provided is a method for hydroponic culture of a plant of the family *Asteraceae* which is suitable for growing the roots of the plant of the family *Asteraceae.* The method for hydroponic culture of a plant of the family *Asteraceae* includes a hydroponic culture step including culturing a plant of the family *Asteraceae* while adjusting the amount of a nutrient solution so that 10 to 50% of the total length from the tip of the tap root of the plant of the family *Asteraceae* is soaked in the nutrient solution, and the rest of the tap root is exposed to a gas.

## Claims

1. A method for hydroponic culture of a plant of the family *Asteraceae,* the method comprising a hydroponic culture step comprising culturing a plant of the family *Asteraceae* while adjusting the amount of a nutrient solution so that 10 to 50% of the total length from the tip of the tap root of the plant of the family *Asteraceae* is soaked in the nutrient solution, and the rest of the tap root is exposed to a gas.

2. The method for hydroponic culture of a plant of the family *Asteraceae* according to claim 1,
wherein the plant of the family *Asteraceae* is *Taraxacum kok-saghyz* or *Taraxacum brevicorniculatum.*

3. The method for hydroponic culture of a plant of the family *Asteraceae* according to claim 1 or 2,
wherein the hydroponic culture step is carried out at a culture temperature of 25°C or lower.

4. The method for hydroponic culture of a plant of the family *Asteraceae* according to any one of claims 1 to 3,
wherein the hydroponic culture step comprises light conditions including a photoperiod of 15 hours or longer and an illuminance at leaf level of 6,000 lx or higher.

## Patentansprüche

1. Verfahren zur hydroponischen Kultur einer Pflanze der Familie *Asteraceae,* wobei das Verfahren einen hydroponischen Kulturschritt umfasst, der das Kultivieren einer Pflanze der Familie *Asteraceae* umfasst, während die Menge einer Nährlösung so eingestellt wird, dass 10 bis 50% der Gesamtlänge von der Spitze der Pfahlwurzel der Pflanze der Familie *Asteraceae* in die Nährlösung eindringt und der Rest der Pfahlwurzel einem Gas ausgesetzt wird.

2. Verfahren zur hydroponischen Kultur einer Pflanze der Familie *Asteraceae* nach Anspruch 1,
wobei die Pflanze der Familie *Asteraceae Taraxacum kok-saghyz* oder *Taraxacum brevicorniculatum* ist.

3. Verfahren zur hydroponischen Kultur einer Pflanze der Familie *Asteraceae* nach Anspruch 1 oder 2,
wobei der hydroponische Kulturschritt bei einer Kulturtemperatur von 25°C oder niedriger durchgeführt wird.

4. Verfahren zur hydroponischen Kultur einer Pflanze der Familie der *Asteraceae* nach einem der Ansprüche 1 bis 3,
wobei der hydroponische Kulturschritt Lichtbedingungen einschließlich einer Photoperiode von 15 Stunden oder länger und einer Beleuchtungsstärke auf Blatthöhe von 6.000 Ix oder höher umfasst.

## Revendications

1. Procédé de culture hydroponique d'une plante de la famille *Asteraceae,* le procédé comprenant une étape de culture hydroponique comprenant la culture d'une plante de la famille *Asteraceae* cependant que la quantité d'une solution nutritive est ajustée de façon que 10 à 50 % de la longueur totale depuis la pointe de la racine pivotante de la plante de la famille *Asteraceae* soient immergés dans la solution nutritive, et le reste de la racine pivotante soit exposée à un gaz.

2. Procédé de culture hydroponique d'une plante de la famille *Asteraceae* selon la revendication 1, dans lequel la plante de la famille *Asteraceae* est *Taraxacum kok-saghyz* ou *Taraxacum brevicorniculatum.*

3. Procédé de culture hydroponique d'une plante de la famille *Asteraceae* selon la revendication 1 ou 2, dans laquelle l'étape de culture hydroponique est mise en œuvre à une température de culture de 25°C ou moins.

4. Procédé de culture hydroponique d'une plante de la famille *Asteraceae* selon l'une quelconque des revendications 1 à 3, dans laquelle l'étape de culture hydroponique comprend des conditions lumineuses comprenant une photopériode de 15 heures ou plus et un éclairement au niveau des feuilles de 6 000 lx ou plus.
